Europäisches Patentamt

European Patent Office

Office européen des brevets

(19)

(11) Publication number: **0 671 170 A1**

(12) **EUROPEAN PATENT APPLICATION**

(21) Application number: 95102428.0

(22) Date of filing: **21.02.95**

(51) Int. Cl.⁶: **A61K 31/20**

(30) Priority: **11.03.94 US 212470**

(43) Date of publication of application:
**13.09.95 Bulletin 95/37**

(84) Designated Contracting States:
**AT BE CH DE DK ES FR GB GR IE IT LI LU MC
NL PT SE**

(71) Applicant: **BRISTOL-MYERS SOUIBB
COMPANY
P.O. Box 4000
Princeton, NJ 08543-4000 (US)**

(72) Inventor: **McGovern, Mark E.
320 South Ouince Street
Philadelphia, PA 19107 (US)**

(74) Representative: **VOSSIUS & PARTNER
Siebertstrasse 4
D-81675 München (DE)**

(54) Use of pravastatin for slowing progression coronary artery atherosclerosis.

(57) Use of pravastatin for the preparation of a pharmaceutical composition for slowing the progression of coronary artery atherosclerosis in patients with proven coronary artery disease and hypercholesterolemia.

The present invention relates to the use of pravastatin for the preparation of a pharmaceutical composition for slowing the rate of progression of coronary artery atherosclerosis, especially in patients with proven coronary artery disease and hypercholesterolemia. The pravastatin is to be administered over prolonged periods.

In accordance with the present invention, surprisingly and unexpectedly, it has been found that patients with coronary artery disease and hypercholesterolemia, who are treated with pravastatin, experience a marked and significant slowing of progression of coronary artery atherosclerosis.

Thus, the present invention relates to the use of pravastatin for the preparation of a pharmaceutical composition for reducing the rate of progression of coronary artery atherosclerosis. The pravastatin is systemically administered to a patient in need of treatment, such as orally or parenterally. Patients to be treated will usually have proven coronary artery disease and/or hypercholesterolemia.

In addition, the present invention relates to the use of pravastatin for the preparation of a pharmaceutical composition for reducing the rate of progression of coronary artery atherosclerosis in man by at least about 40%. The pravastatin is administered in an amount of from about 10 to about 80 mg per day for a sufficient period to reduce rate of progression of coronary artery atherosclerosis by at least about 40%.

Pravastatin, an HMG CoA reductase inhibitor, is disclosed in U.S. Patent No. 4,346,227. As employed herein the term "pravastatin" refers to the free acid form or the form of its salt, preferably pravastatin sodium, available under the trademark Pravacol®.

The term "coronary artery disease" (CAD) as employed herein refers to diseases including atherosclerosis of the coronary arteries, previous myocardial infarction, angina pectoris and/or heart failure.

The term "hypercholesterolemia", as employed herein refers to serum low density lipoprotein-cholesterol (LDL-C) concentrations of $\geq$ 130 and < 190 mg/dL. The patients may also have other risk factors for atherosclerotic coronary artery disease such as high triglycerides, hypertension, previous myocardial infarction, smoker and the like.

It should also be noted that patients to be treated may or may not have proven coronary artery disease and/or hypercholesterolemia as defined above and will still achieve significant slowing of rate of progression of coronary artery atherosclerosis with use of pravastatin in accordance with the present invention.

In carrying our the use of the present invention, pravastatin may be administered to mammalian species, such as dogs, cats, humans, etc., and as such may be incorporated in a conventional systemic dosage form, such as a tablet, capsule, elixir or injectable. The above dosage forms will also include the necessary carrier material, excipient, lubricant, buffer, anti-bacterial, bulking agent (such as mannitol), anti-oxidants (ascorbic acid or sodium bisulfite) or the like. Oral dosage forms are preferred, although parenteral forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing pravastatin in an amount within the range of from about 10 to 80 mg, per day in single or divided doses, and preferably from about 30 to about 50 mg per day in single or divided doses, more preferably about 40 mg per day in a single dose.

A preferred oral dosage form, such as tablets or capsules, will contain pravastatin in an amount of from about 10 to about 80 mg, preferably from about 10 to about 40 mg, and more preferably about 20 mg.

Tablets of various sizes can be prepared, e.g., of about 15 to 2000 mg in total weight, containing the active substance in the ranges described above, with the remainder being a physiologically acceptable carrier or other materials according to accepted pharmaceutical practice. These tablets can, of course, be scored to provide for fractional doses. Gelatin capsules can be similarly formulated.

Liquid formulations can also be prepared by dissolving or suspending active substances in a conventional liquid vehicle acceptable for pharmaceutical administration so as to provide the desired dosage in one to four teaspoonful.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, aspartame, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

The formulations as described above will be administered for a prolonged period, that is, for as long as it is necessary to significantly slow progression of coronary artery atherosclerosis. Sustained release forms of such formulations which may provide such amounts biweekly, weekly, monthly and the like may also be employed. A dosing period of at least one to two months or until rate of progression of coronary artery atherosclerosis has significantly slowed are required to achieve minimal benefit. Administration of the pravastatin formulation may be continued for life to ensure that slowing of progression of coronary artery atherosclerosis continues to be achieved and maintained.

Figure 1 is a schematic diagram of portions of coronary artery (A, B, C and D) showing various quantitative coronary artery evaluation criteria; and

Figure 2 is a graph showing the effects of treatment with pravastatin on mean and minimum coronary lumen diameters and percent stenosis of all subjects with follow-up angiography.

The following Examples represent preferred embodiments of the invention.

Example 1

A pravastatin formulation in the form of tablets having the following composition was prepared as described below.

| Ingredient | Parts by Weight |
|---|---|
| Pravastatin | 7 |
| Lactose | 67 |
| Microcrystalline cellulose | 20 |
| Croscarmellose sodium | 2 |
| Magnesium stearate | 1 |
| Magnesium oxide | 3 |

Pravastatin, magnesium oxide and a fraction (30%) of the lactose were mixed together for 2 to 10 minutes employing a suitable mixer. The resulting mixture was passed through a #12 to #40 mesh size screen. Microcrystalline cellulose, croscarmellose sodium and the remaining lactose were added and the mixture was mixed for 2 to 10 minutes. Thereafter, magnesium stearate was added and mixing was continued for 1 to 3 minutes.

The resulting homogeneous mixture was then compressed into tablets each containing 5 mg, 10 mg, 20 mg or 40 mg pravastatin which may be used in slowing progression of coronary artery atherosclerosis in accordance with the present invention.

Example 2

Pravastatin tablets are prepared employing conventional pharmaceutical techniques containing 20 mg pravastatin and inert ingredients, namely microcrystalline cellulose, providine, lactose, magnesium stearate and croscarmellose sodium as described in the 1994 PDR.

The pravastatin tablets may be employed to slow progression of coronary artery atherosclerosis in accordance with the present invention.

Example 3

This example describes a large national multicenter study entitled "Pravastatin Limitation of Atherosclerosis in the Coronary Arteries (PLAC I)" conducted by 47 investigators at 13 sites in the U.S. The protocol is discussed in Am J. Cardiol. 1993;72:31-35. The study period was from December 29, 1987, through June 23, 1993.

Objectives:

To determine whether treatment with pravastatin could reverse or retard the progression of coronary atherosclerosis and reduce adverse cardivascular sequelae in moderately hypercholesterolemic subjects with coronary artery disease (CAD).

Duration of Treatment:

Subjects were treated for 36 months.

Study Design and Methods:

This was a randomized, double-blind, placebo-controlled, multicenter trial. Subjects undergoing baseline coronary angiography (CAG) were screened for inclusion. Following dietary assessment and counseling, subjects who qualified were randomized (1:1) to either pravastatin 40 mg qd or to matching placebo. The dose was to remain constant for the duration of the study; coronary angiography was repeated at the end of treatment.

Subject Disposition and Demographic Characteristics:

Four-hundred eight (408) subjects were randomized (pravastatin, 206; placebo, 202) and 264 subjects completed 36 months of study therapy including follow-up angiography. The mean age of the randomized subjects (77% men, 23% women) was 56.8 years in the pravastatin and 57.3 years in the placebo group.

Diagnosis and Inclusion Criteria:

Men and postmenopausal or surgically sterile women < 75 years old were eligible for enrollment into the study if they met the following criteria: undergoing coronary angiography after recent myocardial infarction (MI) or for percutaneous transluminal coronary angioplasty (PTCA), provided that the angiography did not reveal only normal coronary arteries; or undergoing diagnostic coronary angiography for chronic or unstable angina that revealed at least one angiographically documented stenosis ≧ 50% in a major coronary artery; serum LDL-C ≧ 130 and < 190 mg/dL and triglycerides (TG) ≦ 350 mg/dL. Subjects with any of the following conditions were excluded: uncontrolled hypertension; endocrine disease; Type III hyperlipoproteinemia; congestive heart failure; debilitating noncardiac chronic disease; significant renal or hepatic disease; chronic pancreatitis; dysproteinemia; porphyria; lupus erythematosus; poorly controlled or insulin-dependent diabetes mellitus; likelihood of coronary artery bypass graft (CABG) surgery or PTCA to the qualifying coronary artery within 6 months; history of cerebrovascular disease; significant gastrointestinal disease; excessive ethanol consumption; hypersensitivity to 3-hydroxy-3-methylglutaryl Coenzyme A (HMG CoA) reductase inhibitors; treatment (which cannot be withdrawn) with corticosteroids, estrogen > 1.24 mg daily, androgens, fish oil, barbiturates, antacids, or other lipid-lowering drugs; or treatment with an investigational drug within 30 days of enrollment.

Test Product:

Pravastatin 20 mg tablets (as per Example 2). Placebo for pravastatin 20 mg tablets.

Criteria for Evaluation:

Efficacy:

The quantitative angiographic efficacy variables included the rate of progression during the interval between baseline and follow-up angiograms in the mean, minimum, and maximum coronary artery lumen diameters, and percent lumen diameter stenosis (%DS) averaged over all available pre-defined coronary artery segments (N ≦ 10 per subject). Any follow-up angiogram obtained > 90 days after randomization was eligible for inclusion in the analysis. Bypassed segments and vessels subjected to PTCA (within 9 months of baseline angiography or subsequent to randomization) were excluded from analysis. The effects of study drug on cardiovascular events and lipid measurements (total cholesterol [Total-C], LDL-C, HDL-C, and TG) were also evaluated. Cardiovascular events were analyzed in two ways: 1) > 90 days after randomization to allow for a maximum effect of pravastatin on serum lipids; and 2) from the time of randomization.

Safety:

Investigators were instructed to document the occurrence of clinical adverse events (AEs; illnesses, signs, or symptoms that had appeared or worsened during the course of the study), both those volunteered by the subjects and those elicited by general questioning, at all scheduled visits. Safety laboratory values were also evaluated periodically during the trial. Other safety tests included a complete medical history and periodic physical examinations, a chest X-ray, and a 12-lead electrocardiogram.

Statistical Methods:

The quantitative angiographic outcome variables, calculated as the rate of progression for mean, minimum, and maximum lumen diameters, and %DS averaged over all available native (unbypassed, non-PTCA) coronary artery segments, were analyzed by an analysis of covariance (ANCOVA) with terms for treatment, site, treatment-by-site interaction, and baseline lumen diameter as the covariate, weighting each site inversely proportional to its variance of the treatment difference. Consistency of results across sites was assessed by testing treatment-by-site interaction terms at a 10% significance level. Clinical cardiovascular events were analyzed by a time-to-event analysis. The mean percent change in lipid levels and selected clinical laboratory analytes were analyzed by ANCOVA. Between-group comparisons of adverse events and marked laboratory abnormalities were assessed by Fisher's Exact test.

Results:

Efficacy:

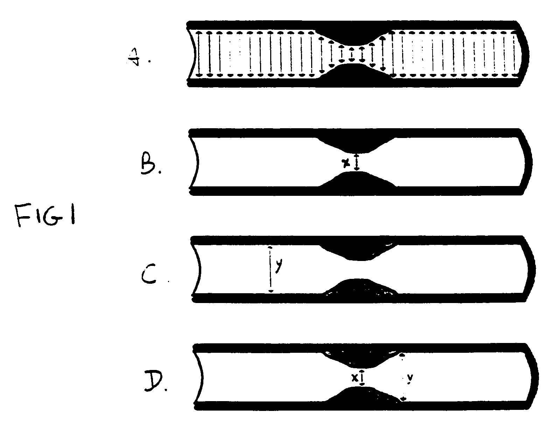

The angiography efficacy variables for the study included the rate of progression in the mean, minimum, and maximum coronary artery diameters and %DS averaged over all available native segments ($N \leq 10$) analyzed (See Fig. 1). Mean diameter of a coronary artery as shown in Fig. 1A, is calculated as

$$\frac{\sum_{1}^{n} i \updownarrow}{n}$$

Fig. 1B shows minimum diameter (x) of a coronary artery.
Fig. 1C shows maximum diameter (y) of a coronary artery.
Fig. 1D shows percent diameter stenosis

$$\left( 1-\frac{x}{y} \right) \times 100\%$$

where y is adjacent normal diameter.

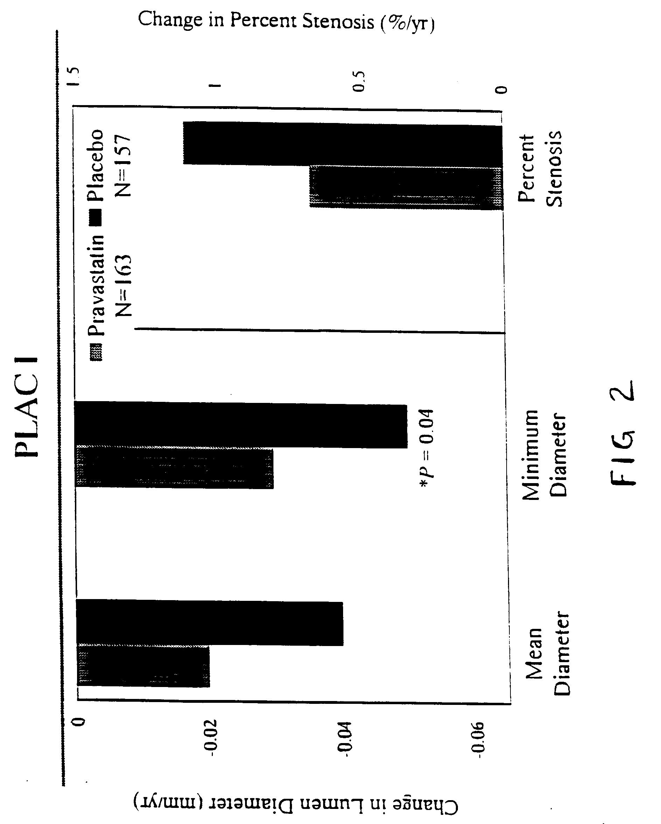

Figure 2 shows the effect of treatment with pravastatin on mean and minimum coronary lumen diameters and percent stenosis for all subjects with follow-up angiography.

As shown in the following Table and Figure 2, narrowing of the lumen of the coronary vessels, as assessed by measurements of mean and minimum lumen diameters and percent lumen diameter stenosis, was attenuated 40%-50% by pravastatin therapy. Changes in maximum lumen diameter (pravastatin = -0.02 mm/yr; placebo = -0.04 mm/yr; P = .20) were consistent with these observations.

Table

| Effects of Treatment on Rate of Change in Coronary Lumen Diameters and Percent Lumen Diameter Stenosis For All Subjects With Follow-up Angiography | | | |
|---|---|---|---|
| Angiographic Endpoint | Pravastatin (N = 163) | Placebo (N = 157) | Between-Group P-Value |
| Neon lumen diameter[a] | -0.02 | -0.04 | 0.16 |
| Minimum lumen diameter[a] | -0.03 | -0.05 | 0.04 |
| Percent lumen diameter stenosis[b] | 0.69 | 1.12 | 0.13 |

[a]Measurement given as mm/year; a negative change indicates a narrowing of the lumen.
[b]Measurement given as %/year; a positive change indicates a narrowing of the lumen.

As shown in the above Table, compared with placebo (N = 157), pravastatin (N = 163) reduced by 40%-50% the rate of progression in mean (pravastatin = -0.02 mm/yr; placebo = -0.04 mm/yr; P = .16) and minimum (pravastatin = -0.03 mm/yr; placebo = -0.05 mm/yr; P = .04) coronary artery lumen diameters and %DS (pravastatin = 0.69 %/yr; placebo = 1.12 %/yr; P = .13). A corresponding reduction was observed in maximum lumen diameter (pravastatin = -0.02 mm/yr; placebo = -0.04 mm/yr; P = .20).

The analysis of clinical cardiovascular events showed a benefit of pravastatin treatment on events that occurred > 90 days after randomization; pravastatin reduced the rates of nonfatal or fatal MI (pravastatin, 2.7%; placebo, 10.5%; P = .006), nonfatal MI or all deaths (pravastatin, 4.4%; placebo, 11.6%; P = .020), and nonfatal MI or coronary heart disease (CHD) death (pravastatin, 3.9%; placebo, 11.0%; P = .016). For events from the time of randomization, comparable effects were observed on the rates of nonfatal or fatal MI ((pravastatin, 4.2%; placebo, 10.5%; P = .0498), nonfatal MI or all deaths (pravastatin, 5.9%; placebo, 12.0%; P = .0720), and nonfatal MI or CHD death (pravastatin, 5.3%; placebo, 11.4%; P = .0652).

For the composite endpoint of nonfatal MI, all deaths, stroke, or PTCA/CABG, pravastatin exerted a blunted effect compared to the other clinical event endpoints (> 90 days after randomization: pravastatin = 18.6%, placebo = 23.9%, P = .249; from time of randomization: pravastatin = 23.3%; placebo = 26.8%, P = .4843). In this event category, for events > 90 days after randomization, 3.9% of subjects in the pravastatin group experienced > one event compared to 9.9% of subjects in the placebo group. Similarly, for events after randomization, 5.3% of subjects in the pravastatin group experienced > one event compared to 10.9% of subjects in the placebo group.

Safety:

Pravastatin was well tolerated during this study. No serious AEs were attributed to pravastatin therapy. Six subjects dies during treatment or less than one month after discontinuation of study therapy, two in the pravastatin group and four in the placebo group. None of the deaths were attributed to study therapy.

Conclusions:

The results demonstrate that pravastatin administration for 36 months to subjects with proven CAD and moderate hypercholesterolemia slowed the progression of coronary artery atherosclerosis and reduced adverse cardiovascular sequelae.

Claims

1. Use of pravastatin for the preparation of a pharmaceutical composition for slowing progression of coronary artery atherosclerosis.

2. The use as defined in Claim 1 wherein said pravastatin is in the form of pravastatin sodium.

3. The use as defined in Claim 1 wherein the patient to be treated has hypercholesterolemia, and proven coronary artery disease.

4. The use as defined in Claim 1 wherein said pravastatin is administered in single or divided doses of from about 15 to about 2000 mg/one to four times daily.

5. The use as defined in Claim 1 wherein the pravastatin is administered in an amount of from about 10 to about 80 mg per day.

6. The use as defined in Claim 1 wherein the pravastatin is administered in an amount of from about 30 to 50 mg per day.

7. The use as defined in Claim 1 wherein the pravastatin is administered in an amount of about 40 mg per day.

8. The use as defined in Claim 7 wherein the pravastatin is administered in a single dose.

9. The use as defined in Claim 1 wherein the patient prior to treatment has serum low density lipoprotein-cholesterol of within the range of $\geq 130$ and $< 190$ mg/dL and triglycerides (TG) $\leq 350$ mg/dL and proven coronary artery disease.

10. The use as defined in Claim 1 wherein rate of progression of coronary artery atherosclerosis is reduced by from about 40 to about 50%

11. A use according to claim 1 for reducing the rate of progression of coronary artery atherosclerosis in man by at least about 40%, wherein the pharmaceutical composition prepared is such that the pravastatin is administered in an amount of from about 10 to about 80 mg per day for a sufficient period to reduce rate of progression of coronary artery atherosclerosis by at least about 40%.

12. The use as defined in Claim 11 wherein the patient to be treated has hypercholesterolemia and coronary artery disease.

FIG 1

PLAC I

Change in Percent Stenosis (%/yr)

Pravastatin N=163    Placebo N=157

*P = 0.04

Mean Diameter    Minimum Diameter    Percent Stenosis

Change in Lumen Diameter (mm/yr)

FIG 2

**European Patent Office**

# EUROPEAN SEARCH REPORT

Application Number

EP 95 10 2428

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | FORTSCHR. MED., vol. 112,no. 4, 20 February 1994 pages 57-64, PRAVASTATIN MULTINATIONAL STUDY GROUP 'Pravastatin bei Patienten mit kardialen Risikofaktoren' *see the whole document * | 1-5,9 | A61K31/20 |
| Y | *  "  * | 6-8, 10-12 | |
| X | BIOCHIMICA ET BIOPHYSICA ACTA, vol. 960,no. 3, 1988 pages 294-302, WATANABE ET AL. 'Preventive effect of pravastatin sodium, a potent inhibitor of 3-hydroxy-3-methylglutaryl coenzym A reductase, on coronary atherosclerosis and xanthoma in WHHL rabbits' * see the whole document * | 1,2 | |
| Y | *  "  * | 6-8, 10-12 | TECHNICAL FIELDS SEARCHED (Int.Cl.6) |
| D,Y | AM. J. CARDIOL. , vol. 72,no. 1, 1 July 1993 pages 31-35, PITT ET AL. 'Design and recruitment in the United States of a multicenter quantitative angiographic trial of pravastatin to limit atherosclerosis in the coronary arteries (PLAC I)' * see the abstract * | 6-8, 10-12 | A61K |

The present search report has been drawn up for all claims

| Place of search | Date of completion of the search | Examiner |
|---|---|---|
| MUNICH | 19 June 1995 | Isert, B |

EPO FORM 1503 03.82 (P04C01)